Europäisches Patentamt

European Patent Office

Office européen des brevets

(11) Veröffentlichungsnummer: **0 287 687**
**A1**

(12) # EUROPÄISCHE PATENTANMELDUNG

(21) Anmeldenummer: 87105686.7

(22) Anmeldetag: 16.04.87

(51) Int. Cl.⁴ **A61N 1/04 , A61N 1/16 , H02N 11/00 , H05F 7/00**

(43) Veröffentlichungstag der Anmeldung:
26.10.88 Patentblatt 88/43

(84) Benannte Vertragsstaaten:
AT BE CH DE ES FR GB GR IT LI LU NL SE

(71) Anmelder: Oehme, Rainer
Bergstrasse 15
D-6415 Petersberg(DE)

(72) Erfinder: Oehme, Rainer
Bergstrasse 15
D-6415 Petersberg(DE)

(74) Vertreter: Schlagwein, Udo, Dipl.-Ing.
Anwaltsbüro Ruppert & Schlagwein
Frankfurter Strasse 34
D-6350 Bad Nauheim(DE)

(54) Vorrichtung zur Behandlung von Lebewesen und Pflanzen durch Pyramidenenergie.

(57) Von einer Pyramide (6) wird Pyramidenenergie mittels eines auf ihr von oben her aufgesetzten Schwingungsaufnehmers abgegriffen. Leitungen (13, 14) sind von diesem Schwingungsaufnehmer (10) jeweils entlang einer Kante der Pyramide (6) herabgeführt und leiten die aufgenommene Energie zu einer oberen und unteren Chakra-Elektrode (8, 9), welche an einem Stativ (7) befestigt ist.

Fig. 1

EP 0 287 687 A1

## Vorrichtung zur Behandlung von Lebewesen und Pflanzen durch Pyramidenenergie

Die Erfindung bezieht sich auf eine Vorrichtung zur Behandlung von Lebewesen und Pflanzen durch Pyramidenenergie, bei der auf der Spitze einer Pyramide ein von oben her über die Pyramide greifender Schwingungsaufnehmer aufgesetzt ist, welcher an jeder Kante der Pyramide und jeder Flächenmittellinie jeweils eine Energieaufnahmeelektrode hat, von der aus insgesamt zwei elektrische Leitungen zu zwei Chakra-Elektroden führen. Eine solche Vorrichtung ist Gegenstand der DE-OS 35 30 841.

Die Tatsache, daß in Pyramiden irgendwelche Energien wirken, die beispielsweise Tiere und Pflanzen mumifizieren, war in Ägypten den Erbauern der Pyramiden schon bekannt. Auch neuere Versuche, über die im Fernsehen berichtet wurde, zeigen, daß sich im Inneren von der Cheops-Pyramide maßstäblich nachgebildeten Pyramiden Lebensmittel länger frisch halten als unter sonst gleichen Bedingungen außerhalb der Pyramide.

Die eingangs genannte DE-OS 35 30 841 zeigt, wie die sogenannte Pyramidenenergie von elektrischen Leitern aufgenommen und zu zwei Chakra-Elektroden geleitet werden kann. Bringt man radioaktiv belastetes Wasser zwischen die beiden Chakra-Elektroden einer Vorrichtung gemäß der genannten DE-OS 35 30 841 und mißt mittels eines Geigerzählers die radioaktiven Impulse, so läßt sich feststellen, daß die Impulszahl innerhalb von zwanzig Minuten von 670 Impulsen pro Minute auf 560 Impulse pro Minute absinkt, während sie bei einer gleichen Wasserprobe außerhalb der Vorrichtung konstant bleibt.

Die vorbekannte Chakra-Elektrodenanlage wird bereits im größeren Umfang zur Behandlung von Menschen eingesetzt. Da die Wirkung der Vorrichtung von der an den Chakra-Elektroden vorhandenen Energie abhängt, ist es wünschenswert, wenn von der Pyramide eine möglichst große Energiemenge aufgenommen werden kann, um entweder eine relativ hohe Energiemenge zur Verfügung zu haben oder aber um mit einer möglichst kleinen Pyramide eine noch ausreichende Energiemenge zur Verfügung stellen zu können.

Der Erfindung liegt die Aufgabe zugrunde, eine Vorrichtung der eingangs genannten Art derart zu gestalten, daß in ihre elektrischen Leitungen eine möglichst große Pyramidenenergiemenge gelangen kann.

Diese Aufgabe wird erfindungsgemäß dadurch gelöst, daß die beiden zu den Chakra-Elektroden führenden elektrischen Leitungen von der Basis der Pyramide entlang zweier Kanten der Pyramide zum Schwingungsaufnehmer hochgeführt sind.

Überraschenderweise hat sich gezeigt, daß Pyramiden an ihren Kanten eine hohe Energie nach außen abstrahlen, die von den auf ihnen entlanggeführten Leitungen aufgenommen werden kann. Versuche mit radioaktiv belastetem Wasser zeigten gegenüber der bekannten Vorrichtung einen deutlich rascheren Abfall der Radioaktivität.

Eine weitere Verbesserung der Energieaufnahme läßt sich erreichen, wenn gemäß einer vorteilhaften Ausgestaltung der Erfindung von jeder zu den Chakra-Elektroden führenden Leitung Einzelleitungen zu jeder Energieaufnahmeelektrode führen und die Einzelleitungen der Leitung zu der einen Chakra-Elektrode im umgekehrten Drehsinn zu den Energieaufnahmeelektroden geführt sind wie die Einzelleitungen der anderen Leitung.

Zur Behandlung von Lebewesen ist es ganz besonders vorteilhaft, wenn die Einzelleitungen zu der Leitung, welche zu der unterhalb des zu behandelnden Lebewesens oder der Pflanze anzuordnenden Chakra-Elektrode führt, von den Energieaufnahmeelektroden im Uhrzeigersinn um die Pyramide herumgeführt sind. Eine solche Anlage wurde ebenfalls mit radioaktiv belastetem Wasser getestet und mit einer Vorrichtung nach dem Stand der Technik verglichen. Es zeigte sich, daß die mit einem Geigerzähler gemessene Impulszahl in der erfindungsgemäßen Vorrichtung innerhalb von 20 Minuten von 670 Impulse auf 400 Impulse pro Minute abfiel, während sie bei der Vorrichtung nach der DE-OS 35 30 841 in der gleichen Zeit nur auf 560 Impulse pro Minute abfiel und bei nicht in einer solchen Vorrichtung eingebrachtem Wasser konstant blieb.

Für die Behandlung von Lebewesen und Pflanzen ist es vorteilhaft, wenn die Einzelleitungen zu der oberhalb des zu behandelnden Lebewesens oder der Pflanze anzuordnenden Chakra-Elektrode im geringeren radialen Abstand zur Pyramide angeschlossen sind, als die zu der anderen Chakra-Elektrode führenden Einzelleitungen. Die positive Wirkung einer solchen Vorrichtung läßt sich deutlich mit Blumen nachweisen. Diese gedeihen zwischen den ChakraElektroden deutlich schneller und besser als außerhalb.

Zur Maximierung der Energiedichte muß die Pyramide mit einer Kante in Nord-Süd-Richtung ausgerichtet werden. Das ist besonders leicht möglich, wenn die Pyramide auf einer Auflage angeordnet ist, welche um eine senkrechte Achse schwenkbar auf einer fahrbaren Grundplatte angeordnet ist, die zugleich ein Stativ zur Halterung der Chakra-Elektroden trägt.

Da Pyramiden auch mit ihrer Grundfläche nach unten hin Energie abstrahlen, ist es zur Nutzung auch dieser Energie vorteilhaft, wenn die Auflage die obere Begrenzung eines Kastens bildet, welcher parallel zur Ebene der Grundfläche der Pyramide im Abstand zu dieser Pyramide einen zur Pyramide hin gerichteten Spiegel aufweist. Ein solcher Spiegel reflektiert die austretende Energie zurück in die Pyramide, wodurch sich der in die Leitungen abgegebene Energieanteil wiederum erhöht. Weiterhin verhindert der Spiegel, daß in einem mehrgeschossigen Gebäude unterhalb der Pyramide befindliche Personen in unerwünschter Weise von der Pyramidenenergie beeinflußt werden.

Besonders wirkungsvoll ist der Spiegel, wenn er von der Grundfläche der Pyramide einen Abstand hat, der mindestens der Höhe der Pyramide entspricht.

Die Handhabung einer Vorrichtung nach der Erfindung ist besonders einfach, wenn das Stativ aus Vierkantrohr besteht und zum Einstecken von Halterungen für die obere und untere Chakra-Elektrode in mehreren, rechtwinklig zueinander stehenden Positionen ausgebildet ist. Mit einem solchen Stativ läßt sich besonders leicht sicherstellen, daß die Elektroden in jeder Gebrauchsstellung miteinander fluchten.

Für die therapeutische Wirkung günstig ist es, wenn die zur oberen Chakra-Elektrode führende Leitung von oben gesehen linksdrehend an der Stativsäule zur oberen Chakra-Elektrode hochgeführt ist.

Aus dem gleichen Grunde vorteilhaft ist eine andere Weiterbildung der Erfindung, gemäß der die zur unteren Chakra-Elektrode führende Leitung von oben gesehen rechtsdrehend zur unteren Chakra-Elektrode geführt ist.

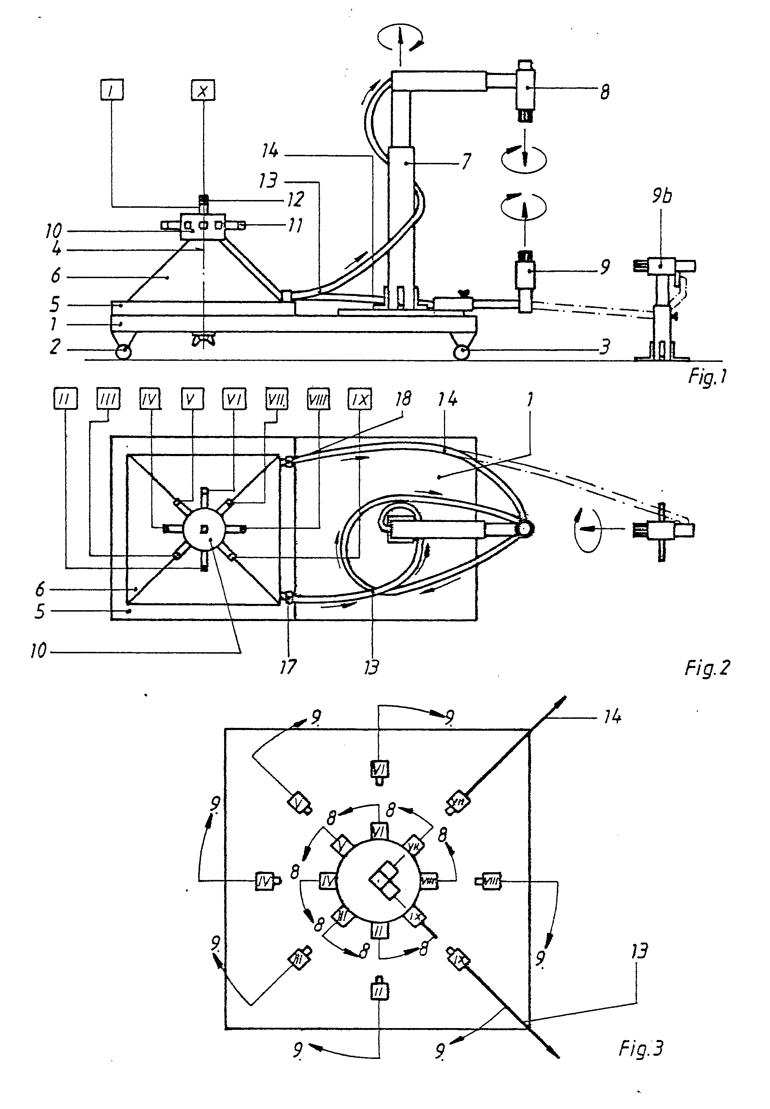

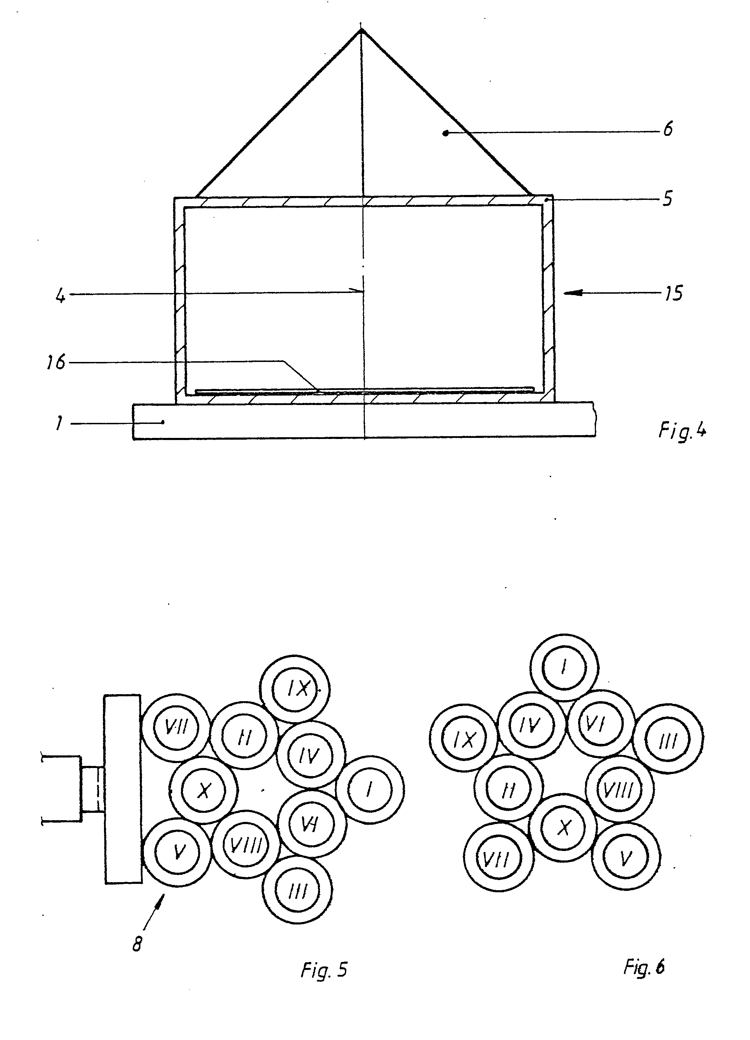

Für die therapeutische Behandlung von Menschen ist es günstig, wenn jede Chakra-Elektrode aus insgesamt zehn parallel zueinander angeordneten Einzelelektroden besteht, welche wie in den Figuren 5 und 6 mit römischen Ziffern gekennzeichnet mit den Energieaufnahmeelektroden gemäß Figur 3 verbunden sind.

Die Erfindung läßt zahlreiche Ausführungsformen zu. Zur weiteren Verdeutlichung ihres Grundprinzips sind zwei davon in der Zeichnung dargestellt und werden nachfolgend beschrieben. Diese zeigt in

Fig. 1 eine Seitenansicht einer erfindungsgemäßen Vorrichtung,

Fig. 2 eine Draufsicht auf die Vorrichtung nach Figur 1,

Fig. 3 eine gegenüber Figur 2 vergrößerte Draufsicht auf die Pyramide mit ihren elektrischen Anschlüssen,

Fig. 4 eine Seitenansicht einer geänderten Ausführungsform.

Fig. 5 eine Ansicht von oben auf die obere Chakra-Elektrode 8

Fig. 6 eine Ansicht von vorn in Richtung des Pfeiles D in Figur 1 auf die Chakra-Elektrode 9b.

Die in Figur 1 dargestellte Vorrichtung hat eine Grundplatte 1, welche als Laufräder 2, 3 aufweisender Wagen ausgebildet ist. Auf dieser Grundplatte 1 ist um eine senkrechte Achse 4 schwenkbar eine Auflage 5 angeordnet, auf der eine Pyramide 6 steht. Durch diese Verschwenk barkeit der Auflage 5 ist es leicht möglich, die Pyramide 6 so auszurichten, daß ihre Grundfläche mit einer Kante in Nord-Süd-Richtung verläuft, was für die Gewinnung einer möglichst großen Pyramidenenergie vorteilhaft ist.

Vor der Auflage 5 ist auf der Grundplatte 1 ein aus Vierkantrohr gebildetes Stativ 7 angeordnet, welches eine obere Chakra-Elektrode 8 und eine untere Chakra-Elektrode 9 haltert. Gestrichelt dargestellt ist eine weitere, untere Elektrode 9b, welche alternativ zur Chakra-Elektrode 9 verwendet werden kann und rechtwinklig zur Chakra-Elektrode 8 ausgerichtet ist.

Von oben her ist auf die Pyramide 6 ein Schwingungsaufnehmer 10 aufgesetzt, welcher auf seiner Mantelfläche insgesamt acht Energieaufnahmeelektroden 11 und koaxial zur Pyramidenspitze auf seiner oberen Stirnfläche eine weitere Energieaufnahmeelektrode 12 aufweist. Jede Energieaufnahmeelektrode 11, 12 hat hintereinander zwei Anschlüsse, von denen jeweils der näher zur Pyramide 6 gelegene Anschluß mit der oberen Chakra-Elektrode 8 und der weiter von der Pyramide entfernt vorgesehene Anschluß mit der unteren Chakra-Elektrode 9 elektrisch verbunden ist.

Die Art der elektrischen Verdrahtung ergibt sich am deutlichsten aus den Figuren 2 und 3. Figur 2 zeigt, daß eine Leitung 13 vom Schwingungsaufnehmer 10 entlang einer Kante der Pyramide 6 nach unten geführt und dann von oben gesehen linksdrehend am Stativ 7 zur oberen Chakra-Elektrode 8 hochgeführt ist. Entsprechend ist eine andere Leitung 14 vom Schwingungsaufnehmer 10 entlang einer anderen Kante der Pyramide 6 nach unten geführt und verläuft ohne die andere Leitung 13 zu kreuzen von oben gesehen rechtsdrehend zur unteren Chakra-Elektrode. Laschen 17, 18 auf der Auflage 5 halten die Lei tungen 13, 14 auf der jeweiligen Pyramidenkante.

In Figur 3 ist zu sehen, daß von den dort mit II bis IX gekennzeichneten, äußeren Anschlüssen der radial nach außen gerichteten Energieaufnahmeelektroden 11 jeweils eine Einzelleitung von oben gesehen im Uhrzeigersinn um die Pyramide 6 herum zur Leitung 14 geführt ist. Wichtig für die Erfindung ist, daß die Energieaufnahmeelektroden nicht unmittelbar miteinander verbunden sind, sondern die Einzelleitungen stets von jeder Energieaufnahmeelektrode ohne Abzweigung zu einer anderen Elektrode zur Leitung 14 geführt sind.

Die in den Figuren 1 und 2 gezeigte Leitung 13, welche zur oberen Chakra-Elektrode 8 führt, ist entsprechend der Leitung 14 mit den Energieaufnahmeelektroden verbunden, wobei der Drehsinn der Einzelleitungen jedoch umgekehrt, also entgegen dem Uhrzeigersinn, verläuft, was in Figur 2 und 3 durch rechteckige Kästchen mit eingetragenen, römischen Ziffern und in Figur 3 zusätzlich durch Pfeile verdeutlicht wurde.

Bei der Ausführungsform gemäß der Figur 4 steht die Pyramide 6 auf einem um die Achse 4 - schwenkbar auf der Grundplatte 1 angeordneten Kasten 15. Im Kasten 15 ist parallel und deckungsgleich zur Basis der Pyramide 6 ein nach oben gerichteter, planer Spiegel 16 angeordnet, dessen Abstand von der Basis der Pyramide 6 zumindest der Höhe der Pyramide 6 entspricht.

Die Figur 5 zeigt, wie zehn mit römischen Ziffern I bis X gekennzeichneten Einzelelektroden der Chakra-Elektrode 8 mit den entsprechend in Figur 3 mit römischen Ziffern gekennzeichneten Energieaufnahmeelektroden verbunden sind. Die untere Chakra-Elektrode 9 ist so mit den Energieaufnahmeelektroden verbunden, daß jeweils ihre mit einer Energieaufnahmeelektrode verbundenen Einzelelek troden mit derjenigen Einzelelektrode fluchten, die mit der gleichen Energieaufnahmeelektrode verbunden ist.

Die Figur 6 zeigt, wie die Einzelelektroden der horizontal ausgerichteten Chakra-Elektrode 9b mit den Energieaufnahmeelektroden zu verbinden sind.

Auflistung der verwendeten Bezugszeichen

1 Grundplatte
2 Laufrad
3 Laufrad
4 Achse
5 Auflage
6 Pyramide
7 Stativ
8 obere Chakra-Elektrode
9 untere Chakra-Elektrode
10 Schwingungsaufnehmer
11 Energieaufnahmeelektrode
12 Energieaufnahmeelektrode
13 Leitung
14 Leitung
15 Kasten
16 Spiegel
17 Lasche
18 Lasche

## Ansprüche

1. Vorrichtung zur Behandlung von Lebewesen und Pflanzen durch Pyramidenenergie, bei der auf der Spitze einer Pyramide (6) ein von oben her über die Pyramide (6) greifender Schwingungsaufnehmer (10) aufgesetzt ist, welcher an jeder Kante der Pyramide (6) und jeder Flächenmittellinie jeweils eine Energieaufnahmeelektrode (11) hat, von der aus insgesamt zwei elektrischen Leitungen (13, 14) zu zwei Chakra-Elektroden (8, 9) führen, dadurch gekennzeichnet, daß die beiden zu den Chakra-Elektroden (8, 9) führenden elektrischen Leitungen (13, 14) von der Basis der Pyramide (6) entlang zweier Kanten der Pyramide (6) zum Schwingungsaufnehmer (10) hochgeführt sind.

2. Vorrichtung nach Anspruch 1, dadurch gekennzeichnet, daß von jeder zu den Chakra-Elektroden (8, 9) führenden Leitung (13, 14) Einzelleitungen zu jeder Energieaufnahmeelektrode (11, 12) führen und die Einzelleitungen der Leitung (13) zu der einen Chakra-Elektrode (8) im umgekehrten Drehsinn zu den Energieaufnahmeelektroden (11, 12) geführt sind wie die Einzelleitungen der anderen Leitung (14).

3. Vorrichtung nach den Ansprüchen 1 oder 2, dadurch gekennzeichnet, daß die Einzelleitungen zu der Leitung, welche zu der unterhalb des zu behandelnden Lebewesens oder der Pflanze anzuordnenden Chakra-Elektrode (9) führt, von den Energieaufnahmeelektroden (11) im Uhrzeigersinn um die Pyramide (6) herumgeführt sind.

4. Vorrichtung nach einem oder mehreren der vorangehenden Ansprüche, dadurch gekennzeichnet, daß die Einzelleitungen zu der oberhalb des zu behandelnden Lebewesens oder der Pflanze anzuordnenden Chakra-Elektrode (8) im geringeren radialen Abstand zur Pyramide (6) angeschlos sen sind, als die zu der anderen Chakra-Elektrode (9) führenden Einzelleitungen.

5. Vorrichtung nach einem oder mehreren der vorangehenden Ansprüche, dadurch gekennzeichnet, daß die Pyramide (6) auf einer Auflage (5) angeordnet ist, welche um eine senkrechte Achse (4) schwenkbar auf einer fahrbaren Grundplatte (1) angeordnet ist, die zugleich ein Stativ (7) zur Halterung der Chakra-Elektroden (8, 9 ) trägt.

6. Vorrichtung nach einem oder mehreren der vorangehenden Ansprüche, dadurch gekennzeichnet, daß die Auflage (5) die obere Begrenzung eines Kastens (15) bildet, welcher parallel zur Ebene der Grundfläche der Pyramide (6) im Abstand zu dieser Pyramide (6) einen zur Pyramide (6) hin gerichteten Spiegel (16) aufweist.

7. Vorrichtung nach einem oder mehreren der vorangehenden Ansprüche, dadurch gekennzeichnet, daß der Spiegel (16) von der Grundfläche der Pyramide (6) einen Abstand hat, der mindestens der Höhe der Pyramide (6) entspricht.

8. Vorrichtung nach einem oder mehreren der vorangehenden Ansprüche, dadurch gekennzeichnet, daß das Stativ (7) aus Vierkantrohr besteht und zum Einstecken von Halterungen für die obere und untere Chakra-Elektrode (8, 9) in mehreren, rechtwinklig zueinander stehenden Positionen ausgebildet ist.

9. Vorrichtung nach einem oder mehreren der vorangehenden Ansprüche, dadurch gekennzeichnet, daß die zur oberen Chakra-Elektrode (8) führende Leitung (13) von oben gesehen linksdrehend an der Stativsäule zur oberen Chakra-Elektrode (8) hochgeführt ist.

10. Vorrichtung nach einem oder mehreren der vorangehenden Ansprüche, dadurch gekennzeichnet, daß die zur unteren Chakra-Elektrode (9) führende Leitung (14) von oben gesehen rechtsdrehend zur unteren Chakra-Elektrode (9) geführt ist.

11. Vorrichtung nach einem oder mehreren der vorangehenden Ansprüche, dadurch gekennzeichnet, daß jede Chakra-Elektrode (8, 9, 9b) aus insgesamt zehn parallel zueinander angeordneten Einzelelektroden besteht, welche wie in den Figuren 5, 6 und 7 mit römischen Ziffern gekennzeichnet mit den Energieaufnahmeelektroden (10, 11) gemäß Figur 3 verbunden sind.

0 287 687

Fig.1

Fig.2

Fig.3

Fig.4

Fig. 5

Fig. 6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 457 792 (HANSCHMANN) * Seite 2, Zeilen 22-41; Figur 6 * --- | 1 | A 61 N 1/04 A 61 N 1/16 H 02 N 11/00 H 05 F 7/00 |
| A | DE-U-8 535 780 (BRÜGEMANN) * Seite 5, Zeilen 1-33; Figuren 1-4 * --- | 3,9,10 | |
| A | DE-A-3 525 521 (KAUSCH) * Spalte 1, Zeile 1 - Spalte 2, Zeile 59; Figuren 1-3 * --- | 1,6,7 | |
| A | EP-A-0 168 513 (RUDHARDT) * Seite 2, Zeile 19 - Seite 3, Zeile 25; Seite 8, Zeile 26 - Seite 10, Zeile 25; Figur 1 * --- | 1,5 | |
| D,A | DE-A-3 530 841 (OEHME) * Seite 8, Zeile 24 - Seite 10, Zeile 14; Ansprüche 10,19; Figur, "Blatt 2" * --- | 1,11 | |
| A | EP-A-0 164 836 (BADGLEY) --- | | |
| A | FR-A-1 153 623 (LARONZE) --- | | RECHERCHIERTE SACHGEBIETE (Int. Cl.4) |
| A | DE-A-3 320 518 (SCHWEITZER) --- | | A 61 N A 23 L H 05 F H 02 N |
| A | FR-A-2 506 533 (CORNILLON) ' --- | | |
| A | FR-A-2 301 941 (GUASCO) ----- | | |

Der vorliegende Recherchenbericht wurde für alle Patentansprüche erstellt

| Recherchenort | Abschlußdatum der Recherche | Prüfer |
|---|---|---|
| DEN HAAG | 10-12-1987 | SCHMIERER U.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes Dokument

EPO FORM 1503 03.82 (P0403)